# EUROPEAN PATENT SPECIFICATION

(11) **EP 3 924 512 B1**
(45) Date of publication and mention of the grant of the patent: **12.07.2023**
(21) Application number: 20705331.5
(22) Date of filing: 11.02.2020
(51) Int. Cl.: C12Q 1/6853, C12Q 1/6806

(54) **PROTOCOLS AND KITS FOR MULTIPLEX AMPLIFICATION AND NGS-SPECIFIC TAGGING**
PROTOKOLLE UND KITS ZUR MULTIPLEX-AMPLIFIZIERUNG UND NGS-SPEZIFISCHEN MARKIERUNG
PROTOCOLES ET KITS POUR AMPLIFICATION MULTIPLEXE ET MARQUAGE SPÉCIFIQUE NGS

(30) Priority: 12.02.2019 EP 19156801
(43) Date of publication of application: 22.12.2021
(73) Proprietor: Biocartis NV, 2800 Mechelen (BE)
(72) Inventor: MEERSSEMAN, Geert, 2800 Mechelen (BE)
(74) Representative: Arnold & Siedsma
(86) International application number: PCT/EP2020/053487
(87) International publication number: WO 2020/165180

(56) References cited:
- US-A1- 2003 148 341
- US-A1- 2018 171 338
- US-A1- 2018 274 029
- PALLAVI SINGH ET AL: "Multilocus sequence typing ofstrains by high-throughput sequencing of selectively amplified target genes", JOURNAL OF MICROBIOLOGICAL METHODS, ELSEVIER, AMSTERDAM, NL, vol. 88, no. 1, 1 November 2011 (2011-11-01), pages 127-133, XP028394699, ISSN: 0167-7012, DOI: 10.1016/J.MIMET.2011.11.004 [retrieved on 2011-11-11]

## Description

### TECHNICAL FIELD

Present invention concerns hairpin-harbouring primer pair mixes and fully automatable single-tube multiplex amplification methods using said primer pair mixes and allowing amplicon tagging, for example with indexes, unique molecular identifiers (UMIs), or other NGS-related tags. In particular, presented herein methods rely on target-specific and universal primer pairs with hairpins having stems comprising a G-T (or G-U) wobble (i.e., as further used herein, anisotropical stems), which allow performing single-tube multiplex PCR amplifications of a potentially large amount of targets. Consequently, present methods and primer pair mixes are particularly suitable for single-tube target enrichment protocols for generating sequencing-ready NGS libraries compatible with various NGS strategies.

### BACKGROUND OF THE INVENTION

Multiplex nucleic acid amplification assays allow simultaneous amplification of several DNA fragments within one reaction. Undoubtedly, this ability to reduce the number of reactions needed to test a sample for multiple different targets helps saving time and resources and makes multiplex systems especially useful in diagnostic setting when large sample and/or target numbers have to be screened. In addition to molecular diagnostics (MDx), e.g. of human genetic variants or infectious agents (*Huang et al. 2018*), multiplex PCR is employed in numerous other fields. For example, they include population genetics and parentage assignment (*Guichoux et al. 2011*), trophic interactions *(Macfadyen et al. 2009),* molecular species identification (*Staudacher et al. 2011*), community assessment (*Gioia et al. 2010*), as well as in forensic (*Hill, Butler & Vallone 2009*) and food safety studies (*Randhawa, Chhabra* & *Singh 2009*). In diagnostic applications, multiplex PCR is most frequently used for direct detection of specific targets thanks to sophisticated chemistries usually based on fluorophore-labelled oligonucleotides (for review cf. *Navarro et al. 2015*), but since more recently it is also becoming more broadly employed as a basis for generation of target-enriched nucleotide libraries for Next Generation Sequencing (NGS) (*Mertes et al. 2011*).

The first use is well known and established in research and clinical practice and there exist excellent automated diagnostic medical devices specialized in delivering timely results based on quantitative real-time PCR (RT-PCR). In order to enable detection of low copy number targets, like rare transcripts or nucleic acids from limited sample sizes, liquid biopsies, or even single cells; many of these devices and existing bench-top assays include in their workflows a so called preamplification step that allows to generate enough specific target molecules for accurate downstream procedures. The multiplex preamplification PCR is usually performed in a limited number of cycles to avoid competition for reagents among the parallel reactions. Furthermore, in order to reduce formation of nonspecific PCR products, the primer concentration applied in it is 10-20 times lower than in a standard PCR, and to compensate for this lower primer concentration, the annealing time is usually extended up to even several minutes. Due to these distinct specific conditions, and also in order to minimize interference from primers and probes used downstream in detection, preamplification PCR is usually performed in a separate tube or compartment than the final detection PCR (*Andersson et al. 2015*). Needless to say, this space-time separation is associated with more laborious workflow and material losses during the transfer of the material between tubes or compartments. Therefore, there exist a need for a robust single tube approach that would combine a highly target-specific multiplex preamplification followed by a robust and unbiased target detection by quantitative real-time PCR.

Concerning uses of multiplex PCR in NGS, it is one of the three major strategies used to achieve targeted enrichment of genome subregions desired for high-resolution sequencing, the other two being target-specific probe-based approaches termed "hybrid capture" and "selective circularization" *(Mertes et al, 2011).* Compared to broader sequencing approaches, such as whole-genome sequencing, targeted enrichment-based NGS is a more cost-effective method for investigating precise areas of interest. It allows clinicians and/or researchers to focus time, expenses, and data analysis on specific nucleic acid regions that are most likely to be involved in the phenotype under study. Consequently, targeted enrichment-based NGS allows for conserving resources and for generating a smaller and more manageable data, and hence also for reducing the final NGS data analysis burden. Targeted approaches also allow to deliver much higher coverage levels; for example, a typical whole genome sequencing usually achieves coverage levels of 30-50× per genome, while a targeted enriched strategy can easily cover the target region at 500-1000× or higher, which allows identification of variants that are rare and too expensive to analyse with whole-genome or Sanger sequencing. Of the three different targeted enrichment strategies, the multiplex PCR-based amplicon sequencing is particularly ideal for analysing single nucleotide variants, insertions/deletions (indels), and rare somatic mutations (e.g. originating from cancerous tumours mixed with germline DNA), as it enables a deep and even coverage over the regions of interest, in addition to providing a more affordable and easier workflow than the probe-hybridisation-based target enrichment methods. Multiplex PCR-based targeted sequencing panels containing targets ranging from a few to hundreds of genes in a single run can be purchased with fixed, preselected content or can be custom designed. According to Illumina's website (status for January 2019), multiplex PCR NGS libraries can be prepared within 5-7.5 hours and sequenced in 17-32 hours.

The major step that poses technical limitation for automation of NGS library preparation via multiplex PCR, and is also a speed limit factor, is the incorporation, usually by ligation, of so called adaptors (or adapters) to the nucleic acids fragments to be sequenced. The adaptors are oligonucleotides of relatively substantial length, that comprise functional sequences of nucleotides or conjugated molecules that are of key importance for a selected NGS platform, strategy, or procedure of choice to be used during sequencing. For example, adaptors for Illumina, Roche 454, Ion torrent, or SOLiD technologies all differ but in simplification it can be stated that an adaptor will usually carry any or several of the following: universal amplification and/or sequencing primer sequences, index/barcode, molecular barcode also known as a unique molecular identifiers (UMI) or another sequence for NGS error reduction. Typically, adaptors are ligated to both ends of nucleic acid fragments, after which the adapter-ligated fragments are then PCR amplified and gel purified. Because of this adaptor-incorporation/ligation step, NGS library preparation from nucleic acid fragments like PCR amplicons, in general comprises at least two steps, in particular because almost always the adaptor sequences carrying the barcodes or UMIs etc. need to be removed before the subsequent amplification. For this reason, the adaptor-containing NGS library preparation is never a single tube procedure. US2018/274029A1 relates to universal hairpin primers.

The present invention addresses this limitation by providing the presented herein primer mixes and amplification methods using hairpins having stems comprising a non-canonical weak G-T (or G-U for RNA) base-pairing, or as further used herein anisotropical hairpins or, in other words, hairpins comprising anisotropical stems. In particular, presented herein methods rely on single-tube mixes of target-specific and universal primer pairs both comprising such anisotropical hairpins, which allow performing single-tube multiplex PCR amplifications of a potentially large amount of targets. Consequently, present methods and primer pair mixes are particularly suitable for single-tube multiplex preamplification followed by quantitative real-time PCR detection, or for single tube generation of adapter-containing sequencing-ready NGS libraries compatible with various NGS strategies. In the latter case, the presence of hairpin allows to easily incorporate the lengthy NGS-strategy specific sequences such as sequencing primers or barcodes into the loops of the hairpins, or for the targets specific primers, the UMIs or other error reduction sequences between the anisotropical hairpin sequence and the target-specific sequence.

The weak G-T or G-U base pairing stabilizes DNA or RNA stems, respectively, mainly through stacking interactions. The reverse complement of a G-T or G-U stabilized stem has C and A at these positions in the helix, which do not pair, interrupt the stacking and do not allow for the reverse complement stem to form at similar melting temperature (Tₘ). We have observed that sequences that form very stable anisotropical hairpins (i.e. comprising the weak G-T or G-U base pairs in their hairpin stems), typically with a Tₘ of around 70°C, can be used as primers, in particular universal primers, for PCRs with annealing step at much lower temperatures. In contrast to these anisotropical hairpins, we also have observed that the "isotropic" hairpins (being, as used herein, hairpins having stems that only contain the canonical G-C and A-T base pairs) with similar Tₘs for both complementary sequences, could not be used as universal primers at lower annealing temperatures. We hypothesise that this is likely because both the isotropical hairpin primer and its complement sequence in the target template are in a hairpin conformation (i.e. have closed stems) at the stated annealing temperature. Therefore, they are unlikely to complementarily pair with each other; in other words, such complements do not 'see' each other in the mixture. With anisotropical stems as universal PCR primers, the primer is in hairpin conformation while the primer-complementary sequence in the template is in linear relaxed conformation (i.e. not closed in a hairpin structure). Hence, in a PCR mix the correct complementary sequence is presented in full and in a relaxed form for annealing with the primer. The stability of the full dsDNA sequence is much higher than the stability of the stem alone. Possibly, through 'breathing' of the hairpin, the more stable full complement is able to form within the timespan of a typical PCR annealing step time.

Herein, we demonstrate that anisotropical hairpins can function as universal primers, while isotropical hairpins with identical Tₘs cannot. We give examples of how this property can be used to append useful sequences, including ones of a typical NGS adapter, into a primer without suffering a PCR-kinetic penalty. We also show how anisotropical target-specific multiplex PCR-suitable primers could enable the use of UMIs or other sequences for NGS error reduction in a single tube PCR, without removal of the primers that carry these sequences and/or without the transfer of tagged sequences to a second tube.

The presented herein primer mixes and methods exploit the thought that a weak G-T base pair can stabilize a stem of a nucleic acid hairpin by more than the base pairing alone but mainly through allowing stacking of the base pairs in the double helix. The reverse complement of the G-T stabilized stem has a cytosine and an adenine at the corresponding positions and therefore a similar weak base pair is not formed. This interrupts the stacking and does not allow for this stem to form at similar Tₘ as its anisotropical-stem forming complement which contains the G-T base pair(s). Based on this phenomenon, we have designed anisotropical hairpin-containing PCR primer appendages, which form hairpins well above the annealing temperature of the PCR. Despite their length, these anisotropical hairpins do not carry kinetic penalties for the primer they are appended to because they do not take part in the hybridization of the 3' end-positioned target-specific primer part to the original template as their DNA is already hybridized in the hairpin stem structure.

As a result of performing initial PCR cycles with such anisotropical-hairpin-appended specific primers, anisotropical-hairpin-appended templates are generated that do not carry hairpin-forming sequences at the annealing temperature. We have observed that these appended templates can function as templates for universal primers that consist solely of anisotropical hairpins at annealing temperatures at which the hairpin is formed. We also demonstrate, as proof of concept, that the same is not true for isotropical hairpins, where both template and universal primer have a hairpin a the priming site at the annealing temperature, precluding the formation of a primer - template structure onto which the DNA polymerase can initiate DNA synthesis during the PCR.

The structure and behaviour of the anisotropical hairpins has a very promising potential for introducing functional sequences such as barcodes into the amplicons generated with primers appended with these anisotropical stems. For example, DNA barcoding methods such as SiMSen-seq (Ståhlberg et al, 2017) or using UMIs (Light et al, 2017) allow lowering of NGS overall error rate for sequencing of PCR amplified input. All molecular barcoding methods require at least a two-step procedure where the primers that carry the UMIs or molecular barcodes are removed from the later amplification.

The presented herein methods based on anisotropical hairpin-appended primers have the potential to introduce barcodes or such error reduction sequences like UMIs in a single tube or chamber without the need of sample transfer or elimination of original primers. Therefore, the presented herein methods allow generation of an adapter-containing NGS-ready library in a single tube protocol that can be easily fully automated, for example in a sample-to-library workflow on an automated platform such as Idylla^{™} of Biocartis NV. To achieve this, gene specific primers with Tₘ of e.g. around 60°C could be appended with UMIs at their 5'-ends and immediately then at the 5' ends of the UMIs, with hairpins bearing anisotropical stems. In such design, the Tₘ of the gene specific primers will remain identical (aside from the very unlikely to impossible, therefore negligible, scenarios wherein a single UMI from the existing very large collection of UMIs would itself anneal with the target gene sequence). These anisotropical hairpin stems from the target specific appended primers would correspond to the anisotropical stems of the hairpins acting as the universal primers designed to anneal at the particular end of the appended amplicon generated with said gene specific appended primers. By the particular end of the appended amplicon it is understood as either referring to the end of the appended amplicon corresponding to the sequence of the forward target specific appended primer, or the end corresponding to the reverse complement of the reverse target specific appended primer, said appended amplicon being generated with these forward and reverse target specific appended primers. Of note, with an assay using a forward and reverse target specific anisotropical-hairpin-stem appended primers, the two anisotropical stems (i.e. one in the reverse and one in the forward appended target-specific primer) have to have different sequences. Consequently, the universal primer anisotropic stems of the forward and reverse universal anisotropic hairpin primers also have to have different sequences (universal forward stem corresponding to the target-specific forward primer stem and the universal reverse stem corresponding to the target-specific reverse primer stem). Otherwise, the appended template generated with said target-specific appended primers may suffer from the so called snap back inhibition, due to having the reverse complement of the first primer present at the other end of each amplicon DNA strand. In such event, it would be required that the UMI-containing primers be removed from the PCR mixture, to avoid their participation in productive events later in the PCR.

Such removal is no longer necessary with anisotropical hairpins for universal primers. First, they can be included in the original mix as they are designed as artificial hairpins not having a full match in the human genome and will therefore be inert in the first 2 (or 3 or 4, depending on the design) cycles of the PCR, as we show herein for different constructs. We have successfully observed full completion of filling in of a template that contains a hairpin at the 3' end (derived from an UMI appended primer) with a commercially available Gotaq polymerase (Promega) without changing cycling conditions as determined for non-appended target specific primer sequences (i.e. for only the target specific part of the appended target-specific primers). This means that fully appended amplicons with one or two UMIs and an anisotropical hairpin containing universal sequences on either of both template ends are formed after cycle 2 when the target is present. To ensure highly efficient single-tube amplification of the thus generated appended templates with universal anisotropical hairpin primers, several measures can be taken to eliminate the long UMI-containing target-specific primers from participation in the subsequent PCR. These include:
1. The concentration of the appended target-specific primers will preferably be lower than the concentration of the two universal primers, in order to ensure amplification of all numerous amplicons (e.g. of a large target enrichment NGS multiplex PCR) at an equal efficiency;
2. Annealing kinetics of the very GC rich anisotropical hairpins to their templates is expected to be favourable compared to the target-specific appended primers, because these will have less extreme 3' ends. Making sure that annealing times and overall cycling protocols are as short as possible with maximize on this advantage;
3. In target-specific appended primers, because the additional NGS-strategy specific sequences like the UMIs are situated just 5' of the target-specific part, there will be mismatching between template and long appended primers in that region, so the annealing of the gene specific part will not be "helped" by an increase in primer - template length. On the other hand, annealed universal primers will readily synthesize over the UMI and target gene area to form full size amplicons;
4. Further handicapping of the long appended target-specific primers can be done to a certain extent by increasing the annealing temperature. For example: in order to ensure that the target-specific part with a Tₘ of e.g. 60°C of the appended target-specific primers is no longer functional, the annealing temperature of the PCR can be increased after 2 or 3 cycles to e.g. 65°C, importantly still being a temperature at which the universal hairpin primers function adequately.

The above scenario would not be possible with isotropical hairpins, as they will not function as primers due to the fact that both primer and template will be in the conformation of a hairpin at the PCR annealing temperature. One could anneal at a much higher temperature after cycle 2 to allow annealing above the Tₘ of the hairpin (e.g. 70°C). However, this is likely to favour nonspecific interactions, as well as lengthen the protocol and overall annealing time, thus reducing the advantage as described in point 2 above; said key desired effect being keeping the long appended primers from making productive primer template hybrids. Furthermore, GC-rich amplicons will be much favoured over AT rich amplicons in such protocols, which is a consideration one may need to avoid where good representation of all amplicons in a large NGS multiplex is a prerequisite. The above scenario is also not possible with universal primers comprising isotropical hairpins, because the annealing would always be hampered by a hairpin-containing template.

Taking into account all of the above listed kinetic considerations, the present invention provides primer mixes and methods and uses based thereupon, that allow single-tube highly advantageous multiplex PCRs with numerous target-specific primer pairs in first several cycles, and then with highly efficient and ensuring uniform amplification of all targets universal primer pair. The above-described and further advantages of the present invention are detailed in the continuation.

### SUMMARY OF THE INVENTION

The present invention is defined in the appended independent claims. Preferred embodiments are defined in the dependent claims. In particular, the present invention concerns a method of thermocycling comprising providing at least two primer pairs in a mix, the first primer pair being a target-specific primer pair and the second pair being a universal primer pair, and subjecting the mix to a thermocycling protocol,
**the method characterised in that**
the target-specific primer pair comprises a target-specific primer comprising
   a target-specific sequence having melting temperature Tₘ₋ₜₛ and
   at a S'-end of said target specific sequence a first anisotropical hairpin defined as having a first loop and a first anisotropical stem comprising at least one G-T or G-U pairing and having melting temperature Tₘ₋ₐₛ₁,
   wherein Tₘ₋ₐₛ₁ is higher than Tₘ₋ₜₛ;
and **in that**
the universal primer pair comprises a universal primer comprising a second anisotropical hairpin defined as having a second loop and a second anisotropical stem comprising at least one G-T or G-U pairing, having melting temperature Tₘ₋ₐₛ₂ and having at least 90% sequence similarity to the first anisotropical stem.

In another aspect the invention concerns a kit comprising a target-specific primer pair and a universal primer pair, the kit characterized in that
the target-specific primer pair comprises a target-specific primer comprising
   a target-specific sequence having melting temperature Tₘ₋ₜₛ and
   at a S'-end of said target specific sequence a first anisotropical hairpin defined as having a first loop and a first anisotropical stem comprising at least one G-T or G-U pairing and having melting temperature Tₘ₋ₐₛ₁,
   wherein Tₘ₋ₐₛ₁ is higher than Tₘ₋ₜₛ;
and **in that**
the universal primer pair comprises a universal primer comprising a second anisotropical hairpin defined as having a second loop and a second anisotropical stem comprising at least one G-T or G-U pairing, having melting temperature Tₘ₋ₐₛ₂ and having at least 90% sequence similarity to the first anisotropical stem.

And finally, in relation to the above, the present invention also concerns the uses of the presented herein methods, primer mixes, and kits in multiplex PCR assays, preferably being part of a diagnostic test or an NGS library preparation protocol.

### BRIEF DESCRIPTION OF FIGURES

For a fuller understanding of the nature of the present invention, reference is made to the following detailed description taken in conjunction with the accompanying drawings in which:
**Figure 1****:** shows two examples of anisotropical hairpins each comprising a stem with two G-T pairings;
**Figure 2****:** shows two examples of regular (i.e. isotropical hairpins) hairpins whose stems contain only regular Watson-Crick base pairing;
**Figure 3****:** shows a melting curves for 4 amplicons (columns) and the rescue of Ct values obtained for low concentrations of target-specific primers by use of anisotropical appended universal primer ("Aniso", top tow) but not by use of isotropical appended universal primer ("Iso", middle row) as compared to non-appended control (bottom row). for experiments performed with

### DEATAILED DESCRIPTION OF THE INVENTION

Present invention generally concerns a method of thermocycling comprising providing at least two primer pairs in a mix, the first primer pair being a target-specific primer pair and the second pair being a universal primer pair, and subjecting the mix to a thermocycling protocol,
**the method characterised in that**
the target-specific primer pair comprises a target-specific primer comprising
   a target-specific sequence having melting temperature Tₘ₋ₜₛ and
   at a S'-end of said target specific sequence a first anisotropical hairpin defined as having a first loop and a first anisotropical stem comprising at least one G-T or G-U pairing and having melting temperature Tₘ₋ₐₛ₁,
   wherein Tₘ₋ₐₛ₁ is higher than Tₘ₋ₜₛ;
and **in that**
the universal primer pair comprises a universal primer comprising a second anisotropical hairpin defined as having a second loop and a second anisotropical stem comprising at least one G-T or G-U pairing, having melting temperature Tₘ₋ₐₛ₂ and having at least 90% sequence similarity to the first anisotropical stem.

As used herein, the term "thermocycling" refers to a nucleic acid template-dependent synthesis procedure, or as used herein "amplification", resulting in an increase in the concentration of the template nucleic acid relative to its initial concentration, wherein the substrates present in the reaction solution, further referred to as the "mix", are repeatedly, i.e. in repeating cycles or "thermocycles", heated and/or cooled to at least two different temperature values. As used herein, the terms "amplification" and "thermocycling" are considered synonyms and their product nucleic acids corresponding to the template nucleic acid will be further referred to as "amplicons". The best known example of thermocycling is a DNA polymerase enzyme-driven synthesis process known as polymerase chain reaction or PCR. DNA polymerase can only synthetize DNA by starting from a free 3'-nucleic acid end. For this reason, PCR requires use of so called primer molecules or "primers" that comprise a sequence sufficiently specific to hybridize to a part of the nucleic acid template sequence as dictated by the well-known rules of complementary base pairing (see, for example, Watson, J. D. et al., In: Molecular Biology of the Gene, 4th Ed., W. A. Benjamin, Inc., Menlo Park, Calif. (1987)). In brief and as well known in the art, PCR "thermocycles" involve repeating cycles of temperature changes that govern the following 3 cycle sub-steps: dissociation of template DNA strands, annealing of at least one primer, and, lastly, extension of the primer by synthesis of amplicons by the DNA polymerase.

The term "nucleic acid" and its equivalent "polynucleotide", as used herein, refer to a polymer of ribonucleotides or deoxyribonucleotides bound together by phosphodiester linkages between the nucleotide monomers. (Deoxy)nucleotides are phosphorylated forms of (deoxy)nucleosides, which most commonly include adenosine, guanosine, cytidine, thymidine, or uridine. These nucleosides consist of a pentose sugar, being ribose or deoxyribose, and a nitrogenous base ("nucleobase", or simply, "base") being either adenine, guanine (that are purines), cytosine, thymine, or uracil (being pyrimidines). The sequence at which these bases (or their nucleosides, or the nucleotides of the latter) follow in a nucleic acid strand is termed "nucleic acid sequence" and is conventionally given in a so called 5'-end to 3'-end direction referring to chemical orientation of the nucleic acid stand. The "S'" originates from the reference to the 5' carbon of the first (deoxy)ribose ring from which the reading of the nucleic acid sequence begins, and the "3"' originates from the 3' carbon of the last (deoxy)ribose ring on which the reading of the nucleic acids sequence ends. A nucleic acid sequences can e.g. be ATATGCC, which is to be interpreted herein as referring to 5'- ATATGCC - 3' nucleic acid sequence. Under the same convention, the latter sequence will be complementary to the sequence 5' - GGCATAT - 3', or simply GGCATAT. The lengths of nucleic acids can simply be given in the number of nucleotides in the sequence or in so called "base pairs" or "bp", one bp corresponding to approximately 3.4 Å (340 pm of length along the nucleic acid strand, and to roughly 618 or 643 daltons for DNA and RNA respectively). Substantial nucleic acid lengths can analogously be described in kilo base pairs = kb (= kbp) = 1,000 bp; mega base pairs = Mb (= Mbp) = 1,000,000 bp, or giga base pairs Gb = 1,000,000,000 bp. Nucleic acids include but are not limited to DNA and RNA, including genomic DNA, mitochondrial or meDNA, cDNA, mRNA, rRNA, tRNA, hnRNA, microRNA, IncRNA, siRNA, and various modified versions thereof. Nucleic acids can most commonly be obtained from natural sources like biological samples obtained from different types of organisms. On the other hand, nucleic acids can also be synthesized, recombined, or otherwise produced in any of the known human-devised methods (e.g. PCR).

As used herein, the term "primer" refers to a nucleic acid of a defined oligonucleotide-level length, that is capable, upon forming a duplex with a polynucleotide template, of acting as a point of initiation of nucleic acid synthesis and being extended from its 3' end along the template so that an extended double strand (duplex) is formed. As used herein, the term "primer pair" refers to at least two such primers, one being termed the "forward primer" and the other one the "reverse primer" complementary to the nucleotide sequences flanking, i.e. being at the beginning and the end of, the section of a desired for amplification part of the template nucleic acid. As used herein, the term "target-specific primer pair" is to be understood as a pair of primers designed to specifically flank and allow for amplification of a unique target sequence, e.g. gene or a fragment thereof, a transcript etc., each primer from said target-specific primer pair comprising a sequence capable of specifically hybridizing with a sequence in their unique target nucleic acid. Conversely, as used herein, the term "universal primer pair" is to be understood as referring to a primer pair designed to hybridize with a non-naturally present synthetic nucleic acid sequence that was introduced in the target-specific primers or in adaptors etc.

As used herein, the term "melting temperature" or "Tₘ" is defined as the temperature at which 50% of double stranded DNA is changed to single-standard DNA. Tₘ depends on the length of the DNA molecule and its specific nucleotide sequence, for example the greater the guanine-cytosine (GC) content, the higher will be the melting temperature of the DNA. In addition, Tₘ calculation also depends on the conditions in which a given dsDNA molecule is, for example its concentration and the concentrations and types of salts present in its solution. Because of these considerations, there is no single standardised formula to calculate Tₘ of a nucleic acid sequence. For example, a popular primer design tool Primer3 uses the oligo melting temperature formula and the table of thermodynamic parameters given in Breslauer et al, 1986, DOI:10.1073/pnas.83.11.3746. Alternative popular approaches base on formulas and parameters as proposed in SantaLucia 1998, DOI:10.1073/pnas.95.4. and von Ahsen et al, 1999, PMID: 10585340. However, there exist many other alternatives for tables of Nearest-Neighbour thermodynamic parameters and for the method of melting temperature calculation. For the purpose of the present disclosure, all the Tₘ values presented herein were computed using a publically available online application for two-state folding nucleic acid analysis from the DINAMelt Web Server of the RNA Institute, University At Albany, State University of New York, using the concentrations of 2 mM for Mg²⁺ and 50 mM for Na⁺. The application bases on the computations as described in Markham & Zuker, 2005, DOI:10.1093/nar/gki591; and in Markham & Zuker, 2008, ISBN 978-1-60327-428-9.

As used herein, the term "hairpin" is to be understood as a typical stem-loop nucleic acid intramolecular structure, frequently seen in various naturally occurring RNAs, which occurs when two regions of the same nucleic acid strand, usually complementary in nucleotide sequence when read in opposite directions, base-pair to form a double helix that ends in an unpaired loop. The paired double helix-comprising part of the hairpin next to the unpaired loop, as used herein, is termed a "stem" or a "hairpin stem". As used herein, a stem comprising only the canonical Watson Crick base pairing (i.e. G pairing with C, and A pairing with T or U), is termed "isotropical stem", while a hairpin built from such isotropical stem is termed herein "isotropical" hairpin. Conversely, as used herein a stem or a hairpin built from a stem comprising at least one non-Watson crick pairing selected from G-T or G-U, will be termed herein as "anisotropical stem" or "anisotropical hairpin", respectively.

In a preferred embodiment, the invention concerns a method of thermocycling comprising providing at least two primer pairs in a mix, the first primer pair being a target-specific primer pair and the second pair being a universal primer pair, and subjecting the mix to a thermocycling protocol,
**the method characterised in that**
the target-specific primer pair comprises a target-specific primer comprising
   a target-specific sequence having melting temperature Tₘ₋ₜₛ and
   at a S'-end of said target specific sequence a first anisotropical hairpin defined as having a first loop and a first anisotropical stem comprising at least one G-T or G-U pairing and having melting temperature Tₘ₋ₐₛ₁,
   wherein Tₘ₋ₐₛ₁ is higher than Tₘ₋ₜₛ;
and **in that**
the universal primer pair comprises a universal primer comprising a second anisotropical hairpin defined as having a second loop and a second anisotropical stem comprising at least one G-T or G-U pairing, having melting temperature Tₘ₋ₐₛ₂ and having at least 90% sequence similarity to the first anisotropical stem, wherein the difference between Tₘ₋ₐₛ₁ and Tₘ₋ₐₛ₂ is not more than 5°C.

Such small difference between Tₘ₋ₐₛ₁ and Tₘ₋ₐₛ₂ is particularly advantageous for the thermocycling protocol design as the second anisotropical stem, in particular its 3'-end is expected to efficiently anneal to the respective part of the complement sequence of the first anisotropical stem being part of the amplicon generated with the target specific primer pair comprising the at least one target-specific primer with the first anisotropical stem.

In a preferred embodiment of the above embodiment, the difference between Tₘ₋ₐₛ₁ and Tₘ₋ₐₛ₂ is not more than 4°C, preferably not more than 3°C, or better not more than 2°C, or even better not more than 1°C, and most preferably wherein Tₘ₋ₐₛ₁ = Tₘ₋ₐₛ₂.

Regardless of differences in existing methods of Tₘ calculation, in a next particularly preferred embodiment, the invention provides a method of thermocycling comprising providing at least two primer pairs in a mix, the first primer pair being a target-specific primer pair and the second pair being a universal primer pair, and subjecting the mix to a thermocycling protocol,
**the method characterised in that**
the target-specific primer pair comprises a target-specific primer comprising
   a target-specific sequence having melting temperature Tₘ₋ₜₛ and
   at a S'-end of said target specific sequence a first anisotropical hairpin defined as having a first loop and a first anisotropical stem comprising at least one G-T or G-U pairing and having melting temperature Tₘ₋ₐₛ₁,
   wherein Tₘ₋ₐₛ₁ is at least 8°C higher than Tₘ₋ₜₛ;
and **in that**
the universal primer pair comprises a universal primer comprising a second anisotropical hairpin defined as having a second loop and a second anisotropical stem comprising at least one G-T or G-U pairing, having melting temperature Tₘ₋ₐₛ₂ and having at least 90% sequence similarity to the first anisotropical stem.

We found that, at least when determining Tm values using the DINAMelt online tool for two-state folding (as described above), the PCRs, at least in our buffering conditions, were performing particularly well when the Tₘ₋ₐₛ₁ was at least 8°C higher than Tₘ₋ₜₛ. However, increasing the difference to at least 9°C, or even better to at least 10°C, could improve the performance even further. Therefore, in preferred embodiments of the method, Tₘ₋ₐₛ₁ is at least 9°C higher than Tₘ₋ₜₛ, or, preferably, Tₘ₋ₐₛ₁ is at least 10°C higher than Tₘ₋ₜₛ.

In a particular embodiment, the invention provides a method of thermocycling comprising providing at least two primer pairs in a mix, the first primer pair being a target-specific primer pair and the second pair being a universal primer pair, and subjecting the mix to a thermocycling protocol,
**the method characterised in that**
   the target-specific primer pair comprises a target-specific primer comprising
      a target-specific sequence having melting temperature Tₘ₋ₜₛ and
      at a S'-end of said target specific sequence a first anisotropical hairpin defined as having a first loop and a first anisotropical stem comprising at least one G-T or G-U pairing and having melting temperature Tₘ₋ₐₛ₁,
      wherein Tₘ₋ₐₛ₁ is at least 8°C higher than Tₘ₋ₜₛ;
   and **in that**
the universal primer pair comprises a universal primer comprising a second anisotropical hairpin defined as having a second loop and a second anisotropical stem comprising at least one G-T or G-U pairing, having melting temperature Tₘ₋ₐₛ₂ and having at least 90% sequence similarity to the first anisotropical stem, wherein the difference between Tₘ₋ₐₛ₁ and Tₘ₋ₐₛ₂ is not more than 5°C.

In line with the above, in particular embodiment of the above embodiment, a method is provided wherein the difference between Tₘ₋ₐₛ₁ and Tₘ₋ₐₛ₂ is not more than 4°C, preferably not more than 3°C, or better not more than 2°C, or even better not more than 1°C, and most preferably wherein Tₘ₋ₐₛ₁ = Tₘ₋ₐₛ₂. In other particular embodiments of the above, a method is provided wherein Tₘ₋ₐₛ₁ is at least 9°C higher than Tₘ₋ₜₛ, or, preferably, Tₘ₋ₐₛ₁ is at least 10°C higher than Tₘ₋ₜₛ.

In an obvious preferred embodiment, a method is provided wherein two primers from the specific primer pair, one being a forward primer and the other being reverse primer, comprise a target-specific sequence and at a S'-end thereof an anisotropical hairpin (i.e. with at least one G-T or G-U pairing). For example, if the target-specific sequence of the forward primer has T_{m-TS} and its anisotropic hairpin stem has Tₘ₋ₐₛ₁, the respective melting temperatures for the reverse primer could be termed T_{m-TS-REV} and T_{m-as1-REV}, respectively. All of these temperatures can be different, it is however important that the difference between any of the target specific sequence melting temperatures T_{m-TS} or T_{m-TS-REV} be as broad as possible with respect to any of the anisotropical hairpin melting temperatures Tₘ₋ₐₛ₁ or T_{m-as1-REV}. The recommended difference is at least 8°C, better 10°C for Tm calculated according to DINAMelt two-state folding application with concentrations of 2 mM for Mg²⁺ and 50 mM for Na⁺ (Markham & Zuker, 2005, DOI:10.1093/nar/gki591; and Markham & Zuker, 2008,ISBN 978-1-60327-428-9).

In a preferred embodiment of the above embodiment, a method is provided wherein two primers from the universal primer pair, one being a forward primer and the other being reverse primer, also comprise an anisotropical hairpin. Naturally, of these anisotropical hairpins, one will be complementary or identical to the anisotropical hairpin of the target specific forward primer, while the other anisotropical hairpin will be complementary or identical to the anisotropical hairpin of the target specific reverse primer. Analogously, if the anisotropic stem of the universal forward primer has Tₘ₋ₐₛ₂, the melting temperature anisotropic stem of the universal reverse primer could be termed T_{m-as2-REV}. As it is advantageous to have tight stems during the first 2 or 3 PCR cycles to keep the universal primers inert and to keep the kinetic ballast of the appended target-specific primers to a minimum, especially later in the PCR protocol, it is also desirable to have the difference between any of the target specific sequence melting temperatures T_{m-TS} or T_{m-TS-REV} be as broad as possible with respect to any of the universal primer anisotropical hairpin melting temperatures Tₘ₋ₐₛ₂ or T_{m-as2-REV}. The recommended difference is at least 8°C, better 10°C for Tm calculated according to DINAMelt two-state folding application with concentrations of 2 mM for Mg²⁺ and 50 mM for Na⁺ (Markham & Zuker, 2005, DOI:10.1093/nar/gki591; and Markham & Zuker, 2008,ISBN 978-1-60327-428-9)

In line with the above considerations, in another embodiment of the invention, a method is provided wherein the second anisotropical stem has at least 95% sequence similarity to the first anisotropical stem, preferably being 98% sequence similarity, most preferably being identical to the first anisotropical stem (or wherein the difference between Tₘ₋ₐₛ₁ and Tₘ₋ₐₛ₂ is not more than 3°C, preferably not more than 1°C, most preferably Tₘ₋ₐₛ₁ is equal to Tₘ₋ₐₛ₂.

As explained above, a given Tₘ of an oligonucleotide will differ for the same oligonucleotide sequence depending on the Tₘ calculation model, conditions specified in the model, and the source of thermodynamic parameters used for the calculation. Therefore, a Tₘ of an oligonucleotide primer sequence or its functional part is merely an indication for selecting the right annealing temperature Tₐ for this oligonucleotide in a PCR protocol. By no means it should be assumed that a given estimated Tₘ will automatically equal the optimal annealing temperature Tₐ to be used during the PCR. Despite the above proviso, in some possible embodiments, a method is provided wherein the thermocycling protocol comprises an annealing step at a temperature Tₐ being at least 6°C, preferably at least 8°C lower than Tₘ₋ₐₛ₁. In another possible embodiment compatible with the above one, a method is provided wherein the Tₐ is not more than 2°C different from Tₘ₋ₜₛ. However, depending on the Tm calculation strategy it can perfectly be normal that for Tₘ₋ₜₛ comprised between 65 and 69°C, the annealing temperature Tₐ in the first initial cycles (i.e. first 2, or first 3, even first 4) will be 64°C.

In another embodiment, a method is provided wherein the temperature difference between Tₐ and Tₘ₋ₜₛ is not more than 2°C, preferably not more than 1°C, most preferably wherein Tₐ is equal to Tₘ₋ₜₛ. In a different embodiment, a method is provided wherein any of Tₘ₋ₜₛ and/or Ta is at least 8°C, preferably 9°C, most preferably 10°C lower than Tₘ₋ₐₛ₁ and Tₘ₋ₐₛ₂.

In a yet another embodiment, a method comparable with the above embodiments is provided, wherein any of Tₘ₋ₜₛ and/or Ta is between 52-66°C, preferably 54-64°C, more preferably is between 56-62°C, most preferably is between around 58-68°C. In a further embodiment of present method, Tₘ₋ₐₛ₁ and preferably Tₘ₋ₐₛ₂ are at least 70°C or higher.

For the reason of sequestering the target-specific primer pair from interfering with the amplification by the universal primer pair, the annealing temperature can be increased after the chosen number of initial cycles. For example, in order to ensure that the target-specific part with a Tₘ of e.g. 60°C of a target-specific primer can no longer anneal, the annealing temperature Tₐ of the PCR can be increased after 2 cycles to a new Tₐ₂ at which the universal still bind and e.g. being 65°C. Hence, in another embodiment a method is provided, wherein the thermocycling protocol further comprises an annealing step at a temperature Tₐ₂, wherein Tₐ₂ > Tₐ. Naturally, given that Tₘ is not always the most reliable predictor for choosing the optimal annealing temperature Tₐ working ranges, mainly due to the above-explained fact that Tₘ can be calculated according to different calculation methods and will differ for the same oligonucleotide depending on the method applied, it is recommended to determine the most robust Tₐ and/or Tₐ₂ experimentally, which is a normal practice for any skilled person.

In a next possible embodiment, for increasing the performance of universal primers in later PCR cycles, a method is provided wherein the concentration of the target-specific primer pair is at least 5 times lower than the concentration of the universal primer pair, preferably being at least 8 times lower, most preferably being at least 10 times lower. It particularly large target-specific multiplex PCR designs according to the present invention, comprising an anisotropical hairpin could be 20 times lower, 50 times lower, or even 100 or 200 times lower.

As explained in the description, a major advantage of the methods of the invention is their suitability for large target number multiplex PCRs. Therefore, in a particularly preferred embodiment, methods are provided wherein the PCR mix comprises at least two, preferably more, like 5, 10, 20, 30, 40, 50, 60, 70, 80, 90, or even 100 or more target-specific primer pairs. In an obvious embodiment of the above embodiment, each of said target-specific primer pairs comprises at least one primer comprising an anisotropical hairpin. In another preferred embodiment, a method is provided, wherein at least one of the at least two or more, preferably of all, target-specific primer pairs comprise a forward primer comprising an anisotropical hairpin and a reverse primer comprising a different anisotropical hairpin having an anisotropical hairpin stem different from the anisotropical hairpin stem of the forward primer. Most preferably, all of the forward target-specific primers will have the same anisotropical hairpin stems. In a further preferred embodiment, all of the reverse target-specific primers will have the same anisotropical hairpin stems but different from the anisotropical hairpin stems of the forward primers. In preferred embodiments of these embodiments, the anisotropical hairpin stems of the target-specific forward primers will be the same as the anisotropical hairpin stem of the universal forward primer, and the anisotropical hairpin stems of the target-specific reverse primers will be the same as the anisotropical hairpin stem of the universal reverse primer, respectively.

In one possible embodiment, the presented herein methods and primer mixes are used in a single-tube multiplex quantitative PCR. The term "quantitative PCR" or simply "qPCR", as used herein, refers to a PCR-based laboratory technique that allows to amplify and simultaneously detect or quantify a targeted nucleic acid molecule. In contrast to standard PCR where the product of the reaction is detected at its end, i.e. after thermocycling has finished, the key feature of qPCR is that the DNA product is being detected during thermocycling as the reaction progresses in "real time"; hence, the alternative name of qPCR "real-time PCR". There currently exist many different types of qPCRs. For example, when starting with a reverse transcription (RT) step, qPCR can be used to quantify numbers of messenger RNAs and is then called a reverse transcriptase qPCR or an RT-qPCR. As used herein the terms "quantitative PCR" or simply "qPCR" will be employed with preference over the term "real-time PCR" or "RT-PCR" in order to avoid confusion with reverse transcription PCR, also frequently abbreviated as RT-PCR. Most qPCRs use one of the two most common methods for detecting the product amplification in real-time: (a) intercalation of non-specific fluorescent dyes with any doublestranded DNA, or (2), which is the one applicable to the methods of the invention, sequence-specific DNA probes consisting of oligonucleotides that are labelled with a fluorescent reporter which permits detection only after hybridization of the probe with its complementary target sequence. The fluorescent signals generated during thermocycling are detected by an appropriate optical detection system and tracked from the moment they pass the background threshold until the reaction reaches a plateau. The copy number of the target sequences can be estimated using either relative or absolute quantification strategy, typically by analysing the shape of the obtained amplification curve (standard curve strategy) or by determining when the signal rises above some threshold value (often called the Ct value, but sometimes also Cp value or Cq value). In relative quantification, the target nucleic acid levels estimated in a given sample using the Ct or standard curve analysis are expressed as relative to values obtained for the same target in another reference sample, for example, an untreated control sample. Conversely, in absolute quantification the qPCR signal is related to input copy number using a standard curve or can also be calculated according to a more recent digital PCR method. For the moment being, the first strategy is still more prevalent and bases the estimation of the target DNA amount by comparing the obtained values with a previously made standard curve. These and other qPCR quantification strategies are broadly known in the art and their calculation can differ depending on a given application and a qPCR system.

In a particularly preferred embodiment, due to the potential of including NGS-specific sequences in the primers, the presented herein methods can be used in targeted enrichment NGS library preparation. As explained before, a full-blown NGS data analysis of whole or substantial part of human genome or exome is currently still a complex endeavour that requires a skilled Bio-IT team. Naturally, this is associated with time constraints and additional costs. In addition, such analyses are still only possible for large expert centres and are frequently for practical reasons inaccessible to smaller laboratories. Especially that the outcomes of such high resolution analyses are full of information, large part of which has no practical relevance for a pathologist and the doctor who has to decide about treatment options for a given patient. Therefore, from clinical perspective and having time-to-treatment considerations in mind, targeted enrichment of genome subregions is of much greater interest for health practitioners and diagnostic laboratories, especially where only a limited number of hotspots or drug-targetable genetic lesions need to be analysed. By selective recovering and subsequent sequencing of loci of interest, costs, staff efforts, and time of diagnosis can substantially be reduced compared with the whole-genome approach. For these and other reasons, in another preferred embodiment of the invention, a method is provided resulting in NGS-ready amplicons serving as a sequencing library. As used herein, the term "sequencing library" or "nucleic acid sequencing library" refers to a set of a plurality of amplified polynucleotides, most often of DNA type, that are meant to be subjected to sequence analysis using any of the currently known nextgeneration sequencing (NGS) strategies. In line with this, in a currently preferred embodiment of the invention, a sequencing library is composed of a plurality of PCR-amplified DNA molecules, most preferably fused or ready to be fused with adapter molecules (or adapter sequences) compatible with a given NGS strategy of choice. A comprehensive overview of sequencing library types and ways of preparing them can be found in a review of van Dijk et al. Exp Cell Res. 2014.

In a possible, embodiment of the above embodiment, the NGS-ready amplicons comprise a clinically relevant NGS panel. Such panels are known in the art and can be designed to target any physiological or pathological condition or state. Some examples include NGS panels specific for diagnosis of a genetic disease, identification or a post-treatment follow up of a cancer, genetic checkup of a foetus during pregnancy, characterisation of a microbiome or of an infectious agent etc. The NGS panels can be of any size depending on the desired coverage. Their amplification can be designed as a single multi-target multiplex or can involve several parallel multiplexes. For example, a panel may be designed as encompassing from 10 to 100, 200, or more target amplicons in a single multiplex reaction (i.e. be a single, 10-plex, 100-plex, or 200-plex, respectively), or can be divided between two or three or more multiplexes, e.g. one 50-plex, one-75-plex and one 99-plex, depending on the need and design constraints such as interaction interactions between primers in a single multiplex reaction. A possible example of the invention could involve comprising two distinct clinically relevant NGS panels, e.g. one directed to cancer and another to inflammation.

As explained above, for the NGS-ready amplicons to be readily portable onto an NGS platform of choice, the fragments destined for sequencing need to comprise certain NGS-platform specific sequences. These include e.g. "indexes" also known as "sample barcodes" or, simply, "tags", which are unique DNA sequences attached to fragments within a sequencing library for downstream *in silico* sorting and identification. Indexes are typically a component of adapters or PCR primers and, in existing in the art methods need to be ligated to the library fragments during the sequencing library preparation stage. Illumina indexes are typically between 8-12bp. Libraries with unique indexes can be pooled together, loaded into one lane of a sequencing flow cell, and sequenced in the same run. Reads are later identified and sorted via bioinformatic software. Altogether, this grouping process is commonly known as "multiplexing", the term will not be used herein in order to avoid confusion with multiplex PCR which is a single tube PCR targeting many targets using distinct target-specific primers.

A different type of an NGS-strategy specific sequence is a "molecular barcode" also known as a "unique molecular identifier" or "unique molecular index" or, shortly, "UMI". UMIs are random oligonucleotide barcodes that have been used to confidently identify PCR duplicates in highthroughput sequencing experiments (König et al. 2010; Kivioja et al. 2012; Islam et al. 2014). By incorporating a UMI into the same location in each fragment during library preparation, but prior to PCR amplification, it is possible to accurately identify true PCR duplicates because they have both identical alignment coordinates and identical UMI sequences. In addition to their use in single-cell RNA-seq and iCLIP (König et al. 2010), UMIs may be applied to almost any sequencing method in which confident identification of PCR duplicates is required. Barcodes and molecular barcodes/UMIs are not mutually exclusive, and both can be necessary for an NGS experiment. This is because UMIs do not participate in grouping samples. Instead, their purpose is to correct a previously tricky problem in the use of PCR during NGS workflows, such as for targeted enrichment of specific regions prior to sequencing, or for library amplification to obtain sufficient material for quantification. PCR amplification inherently leads to errors such as amplification bias, and without a control, the reads ratio can provide skewed data, leading to incorrect conclusions. The other advantage of molecular barcodes is that PCR artefacts can sometimes be misread as sequence variants. This makes identifying low-frequency variants difficult, because one cannot be sure whether the observed difference is a true variant or not. With molecular barcodes, if the majority of the reads for a particular molecule do not contain the "variant", the observed different is an artefact. Conversely, if all of the reads do contain it, even if it is present at a low frequency, one can be confident the variant is real. For these reasons, molecular barcoding / UMIs are extremely important for accuracy in NGS experiments involving PCR.

As barcodes and UMIs are not non-exclusive, in fact they may be included in different primer pairs of the presented herein methods. In an exemplary embodiment, a method is provided wherein the universal primer pair comprises a barcode or a tag or another NGS-strategy specific sequence to fit kits of NGS manufacturers. An example of the latter can be any of the P5/P7 ends for Illumina libraries. In principle, any sequences required for general cell-flow amplicon processing in the sequencing workflow can be incorporated into the loop of the anisotropic hairpins in the universal primer pair. In theory, it would be possible to include NGS sequences of practical use at the 5' of the hairpin, but given the functional lengths of sequencing primer sequences and indexes, it appears more advantageous to constrain the added extraneous DNA into the loop part of the anisotropic hairpins. Such hiding of the NGS useful sequences in the anisotropical hairpins would minimize their crosstalk with the target templates and/or primers within the PCR mix. As the loops of the anisotropic hairpins can in principle be designed to comprise internal hairpins, the presented herein primers have enormous potential for carrying even very long and complex NGS-strategy specific sequences, which in the known method of the art have to in most cases be ligated in the form of adaptors.

Although the above-listed sequences may equally well be included in the target specific primer anisotropic hairpins, the situation looks different for UMIs, which in fact should only be included in the target-specific primers. According to the methods of the invention, the target-specific primers will normally only participate in the PCR reaction during the very initial cycles, for example cycle 1 and 2, possibly 3 and not beyond 4, afterwards being out-competed by the universal anisotropic hairpin primer pairs. Furthermore, UMIs should not be incorporated into the hairpin for the reason of the latter being universal and not unique. Instead, UMIs and other NGS-corrective sequences will more advantageously be positioned between the anisotropic hairpin and the 3'-end positioned target-specific sequence. Thus, in a preferred embodiment a method is provided wherein the target-specific primer further comprises a unique molecular identifier (UMI) or a molecular barcode sequence at the S'-end of the target-specific sequence. In other words, the position concerned is at the S'-end of the gene-specific sequence and at the 3'-end of the first anisotropical hairpin, i.e. between the first anisotropical hairpin and the target specific sequence. The aim of such positioning is to ensure that the sequence designed to add a unique molecular identification to the original target molecule, at the first time of replication of the input target molecule, is placed between the target-specific primer sequence and the universal primer, in this case, an anisotropical hairpin-forming sequence.

In a further preferred embodiment, a method is provided wherein both the reverse and the forward primer of the target specific primer pair comprise a UMI (aka molecular barcode) sequence. In principle, one target molecule could be labelled with only one UMIs, but it is generally betterto have two because (a) pair-end sequencing strategies are becoming a standard in NGS; and (b) it ensures a break in the DNA helix of appended UMI containing template with target specific UMI containing primer, because the two UMIs will not match. For the latter consideration, the lack of UMI equals having to deal with a full dsDNA complement with a much higher stability than one with a 5 to 10 nt double wobble. Said destabilisation can be used in the advantage of designing a robust multiplex PCR in which after the first two cycles the universal primers are expected to outperform the primer-specific ones. One could increase annealing temperature, e.g. starting from cycle 3 to make priming by the substantially longer target-specific primers "lose" to universal primers, which will anneal easily.

As the described above methods can be performed in a single tube or compartment, and therefore can be easily automated, in a naturally preferred embodiment, the method of the invention is performed by an automated system. As used herein, the term "automated system" is to refer to integrated platform comprising an instrument and disposable material, such as plastics and solutions, which the system uses in an automated manner to complete a certain process. Such process can be initiated by a user but throughout its automated processing within the system, the user's intervention is not necessary until the process completion. As used herein the term "instrument" is to be understood as a machine equipped with at least a user interface (e.g. comprising at least a start button or an electricity plug), an onboard computer with software, and programmed to perform certain functions like run an assay, which can e.g. involve mixing, sonication, heating, data detection and collection, and possibly analysis etc. In a possible embodiment, the interface can be in a form of a console comprising a computer system running user interface software capable of initiating tests, displaying test results, and communicating with external information systems. An excellent automated system capable of readily accommodating the methods of the invention is a diagnostic platform Idylla^{™} manufactured by Biocartis NV, which uses a disposable reagent-bearing cartridge that is engageable with a cartridge-processing instrument and provides sample-to-result analytical performance. Along these lines, in a preferred embodiment, the disposable material is provided in a form of cartridge or a kit that comprises a cartridge. As used herein, the term "kit" is to be interpreted as a set comprising at least one article or an assembly or articles or equipment needed for a specific purpose, for example being performing of a molecular biology process or an assay. A kit may comprise a cartridge or simply be provided in a form of a cartridge.

In a preferred embodiment of the above the embodiment, the cartridge of a type that directly accepts a biological sample, obtains a PCR-grade (or as used herein "amplifiable") nucleic acid from it, and is suitable and adapted to house at least one PCR reaction. As used herein, the term "sample" is intended to include a variety of sources of biological origin that contain nucleic acid and/or cellular material containing nucleic acid. The examples of the latter include cultures of cells; usually being eukaryotic cells such as mammalian cells but can be prokaryotic cells such as bacteria. A sample may include body fluids, body fluid precipitates, lavage specimen, fine needle aspirates, biopsy or liquid biopsy samples, tissue samples, cancer cells (fresh or fixed), forensic samples, or any other types of nucleic acid containing material obtained from a patient or an individual being tested and/or treated for presence of a disease or an infection. In general, as used herein the term "sample" is to be interpreted as any source of a nucleic acid suitable to be subjected to an amplification procedure. Along these lines, a "sample" may be an almost intact biological sample readily subjectable to an amplification procedure, such as e.g. a bacterial colony in a colony PCR. More frequently, however, the term "sample" will refer to a nucleic acid-containing result of a biological sample processing procedure such as a biological sample liquefact comprising amplification-ready released nucleic acids or a solution of readily amplifiable nucleic acids purified from a biological sample.

Finally, in a highly advantageous embodiment, a method of the invention is provided wherein the at least two primer pairs are provided within a cartridge engageable with an automated platform. As used herein, the term "cartridge" is to be understood as a self-contained assembly of chambers and/or channels, which is formed as a single object that can be transferred or moved as one fitting inside or outside of a larger instrument that is suitable for accepting or connecting to such cartridge. A cartridge and its instrument can be seen as forming an automated system, further referred to as an automated platform. Some parts contained in the cartridge may be firmly connected whereas others may be flexibly connected and movable with respect to other components of the cartridge. Analogously, as used herein the term "fluidic cartridge" shall be understood as a cartridge including at least one chamber or channel suitable for treating, processing, discharging, or analysing a fluid, preferably a liquid. An example of such cartridge is given in WO2007004103. Advantageously, a fluidic cartridge can be a microfluidic cartridge. In the context of fluidic cartridges the terms "downstream" and "upstream" can be defined as relating to the direction in which fluids flow in such cartridge. Namely, a section of a fluidic path in a cartridge from which a fluid flows towards a second section in the same cartridge is to be interpreted as positioned upstream of the latter. Analogously, the section to which a fluid arrives later is positioned downstream with respect to a section which said fluid passed earlier. In general, as used herein the terms "fluidic" or sometimes "microfluidic" refers to systems and arrangements dealing with the behaviour, control, and manipulation of fluids that are geometrically constrained to a small, typically sub-millimetre-scale in at least one or two dimensions (e.g. width and height or a channel). Such small-volume fluids are moved, mixed, separated or otherwise processed at micro scale requiring small size and low energy consumption. Microfluidic systems include structures such as micro pneumatic systems (pressure sources, liquid pumps, micro valves, etc.) and microfluidic structures for the handling of micro, nano- and picolitre volumes (microfluidic channels, etc.). Exemplary fluidic systems were described in EP1896180, EP1904234, and EP2419705 and can accordingly be applied in certain embodiments of the presented herein invention. In line with the above, the term "chamber" is to be understood as any functionally defined compartment of any geometrical shape within a fluidic or microfluidic assembly, defined by at least one wall and comprising the means necessary for performing the function which is attributed to this compartment. Along these lines, "amplification chamber" is to be understood as a compartment within a (micro)fluidic assembly, which suitable for performing and purposefully provided in said assembly in order to perform amplification of nucleic acids. Examples of an amplification chamber include a PCR chamber and a qPCR chamber.

In another aspect the invention concerns a kit comprising a target-specific primer pair and a universal primer pair, the kit characterized in that
the target-specific primer pair comprises a target-specific primer comprising
   a target-specific sequence having melting temperature Tₘ₋ₜₛ and
   at a S'-end of said target specific sequence a first anisotropical hairpin defined as having a first loop and a first anisotropical stem comprising at least one G-T or G-U pairing and having melting temperature Tₘ₋ₐₛ₁,
   wherein Tₘ₋ₐₛ₁ is higher than Tₘ₋ₜₛ;
and **in that**
the universal primer pair comprises a universal primer comprising a second anisotropical hairpin defined as having a second loop and a second anisotropical stem comprising at least one G-T or G-U pairing, having melting temperature Tₘ₋ₐₛ₂ and having at least 90% sequence similarity to the first anisotropical stem.

In particularly advantageous embodiments, a kit is provided wherein the difference between Tₘ₋ₐₛ₁ and Tₘ₋ₐₛ₂ is not more than 5°C, preferably not more than 4°C, more preferably not more " than 3°C, or better not more than 2°C, or even better not more than 1°C, and most preferably wherein Tₘ₋ₐₛ₁ = Tₘ₋ₐₛ₂. In other particular embodiments of the above, a method is provided wherein Tₘ₋ₐₛ₁ is at least 8°C higher than Tₘ₋ₜₛ, or preferably, Tₘ₋ₐₛ₁ is at least 9°C higher than Tₘ₋ₜₛ, or more preferably, Tₘ₋ₐₛ₁ is at least 10°C higher than Tₘ₋ₜₛ.

In line with the above considerations, in a particularly preferred embodiment, the provided herein kit comprises a cartridge engageable with an automated platform. Naturally, in a preferred embodiment, the target specific and the universal primer pairs are provided within said cartridge, preferably in a spotted form, possibly present within one spot.

In a related aspect, the present invention also provides a cartridge engageable with an automated platform, said cartridge comprising a target-specific primer pair and a universal primer pair, the cartridge characterized in that
the target-specific primer pair comprises a target-specific primer comprising
   a target-specific sequence having melting temperature Tₘ₋ₜₛ and
   at a S'-end of said target specific sequence a first anisotropical hairpin defined as having a first loop and a first anisotropical stem comprising at least one G-T or G-U pairing and having melting temperature Tₘ₋ₐₛ₁,
   wherein Tₘ₋ₐₛ₁ is higher than Tₘ₋ₜₛ;
and **in that**
the universal primer pair comprises a universal primer comprising a second anisotropical hairpin defined as having a second loop and a second anisotropical stem comprising at least one G-T or G-U pairing, having melting temperature Tₘ₋ₐₛ₂ and having at least 90% sequence similarity to the first anisotropical stem.

In particularly advantageous embodiments, a cartridge is provided wherein the difference between Tₘ₋ₐₛ₁ and Tₘ₋ₐₛ₂ is not more than 5°C, preferably not more than 4°C, more preferably not more than 3°C, or better not more than 2°C, or even better not more than 1°C, and most preferably wherein Tₘ₋ₐₛ₁ = Tₘ₋ₐₛ₂. In other particular embodiments of the above, a method is provided wherein Tₘ₋ₐₛ₁ is at least 8°C higher than Tₘ₋ₜₛ, or preferably, Tₘ₋ₐₛ₁ is at least 9°C higher than Tₘ₋ₜₛ, or more preferably, Tₘ₋ₐₛ₁ is at least 10°C higher than Tₘ₋ₜₛ.

Lastly, in a particularly preferred embodiment, the provided herein kit comprises a cartridge engageable with an automated platform. In a preferred embodiment, the

And finally, in relation to the above-described embodiments, the present invention also concerns the uses of the presented herein methods, primer mixes, and kits in multiplex PCR assays, preferably being part of a diagnostic test or an NGS library preparation protocol.

### EXAMPLE

The following experiment shows that universal anisotropical hairpin primers can be used as universal primers for correctly appended amplicons, in a one tube assay, with homogeneous annealing temperatures.

From a large NGS multiplex (>70-plex) assay developed in-house, we first selected 4 primer pairs for generating 4 amplicons of around 100 bp from human DNA material and annealing at 64°C for 30 seconds. The primer pairs are specific for parts of BRAF, KRAS, and two positions within ERBB2 gene. They are respectively labelled BRAF, KRAS, ERBB2a, and ERBB2b. Each of the target amplicon primers was appended with an anisotropical or an isotropical hairpin-bearing sequence that corresponds to a universal hairpin primer.

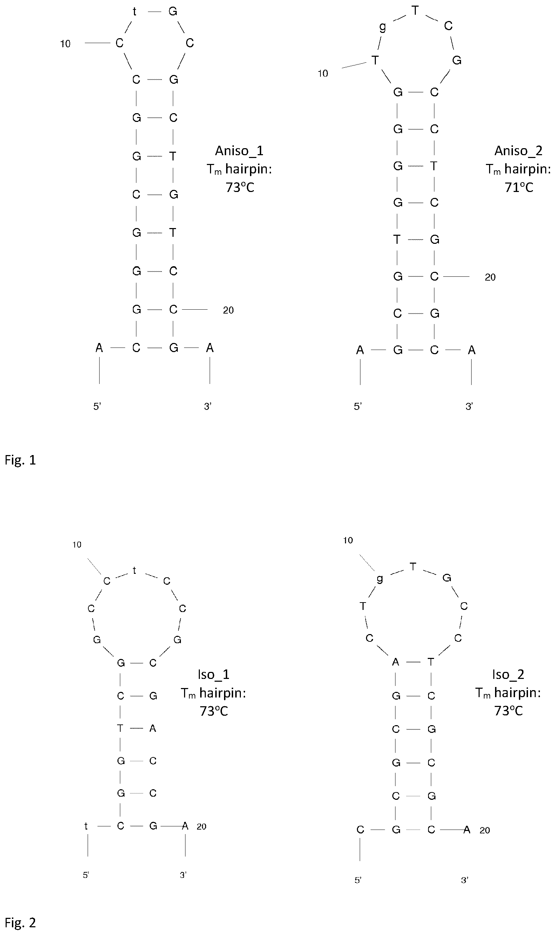

At the chosen annealing temperature of 64°C, all of the universal hairpin primers are in the hairpin formation. The nomenclature is "Aniso" for anisotropical hairpin sequences and "Iso" for isotropical hairpin sequences. The hairpins are shown in Figures 1 and 2, respectively, and have the following sequences and melting temperatures characteristics (Tₘs).

| | | | | |
|---|---|---|---|---|
| Aniso_1 | ACGGGCGGCCtGCGCTGTCCGA | Tₘstem: 73 | Tₘstem-rev-comp: 54 | Tₘ: 84 |
| Aniso_2 | AGCGTGGGGTgTCGCCTCGCGCA | Tₘstem: 71 | Tₘstem-rev-comp: 61 | Tₘ: 84 |
| Iso_1 | tCGGTCGGCCtCCGCGACCGA | Tₘstem: 73 | Tₘstem-rev-comp: 72 | Tₘ: 82 |
| Iso_2 | CGCGCGACTgTGCCTCGCGCA | Tₘstem: 73 | Tₘstem-rev-comp: 73 | Tₘ: 83 |

The bases participating in anisotropical pairing are marked in bold in the above sequences of Aniso_1 and Aniso_2.

The sequences of the amplicons comprising correctly appended anisotropical hairpin primers are shown below. The appended anisotropical target-specific primer sequences are underlined.

BRAF aniso_appended amplicon (132 bp): ACGGGCGGCCtGCGCTGTCCGATGGGCAGATTACAGAGGGA
CAAGAATTGGATCTGGATCATTTGGAACAGTCTACAAGGGAAAGTGGCATGGTAAGTATGTAATGTGGTGCG
CGAGGCGAcACCCCACGCT

KRAS aniso_appended amplicon (139 bp): ACGGGCGGCCtGCGCTGTCCGATGATGGAGAAACCTGTCTCT
TGGATATTCTCGACACAGCAGGTCAAGAGGAGTACAGTGCAATGAGGGACCAGTACATGAGGACTGGGGAG
GGCTGCGCGAGGCGAcACCCCACGCT

ERBB2a aniso_appended amplicon (143 bp): ACGGGCGGCCtGCGCTGTCCGATGTGGCTACATGTTCCTGA
TCTCCTTAGACAACTACCTTTCTACGGACGTGGGATCCTGCACCCTCGTCTGCCCCCTGCACAACCAAGAGGTG
ACAGCTGCGCGAGGCGAcACCCCACGCT

ERBB2b_aniso appended amplicon (143 bp): ACGGGCGGCCtGCGCTGTCCGACCATACCCTCTCAGCGTA
CCCTTGTCCCCAGGAAGCATACGTGATGGCTGGTGTGGGCTCCCCATATGTCTCCCGCCTTTCTGGGCATCTGC
CTGACATGCGCGAGGCGAcACCCCACGCT

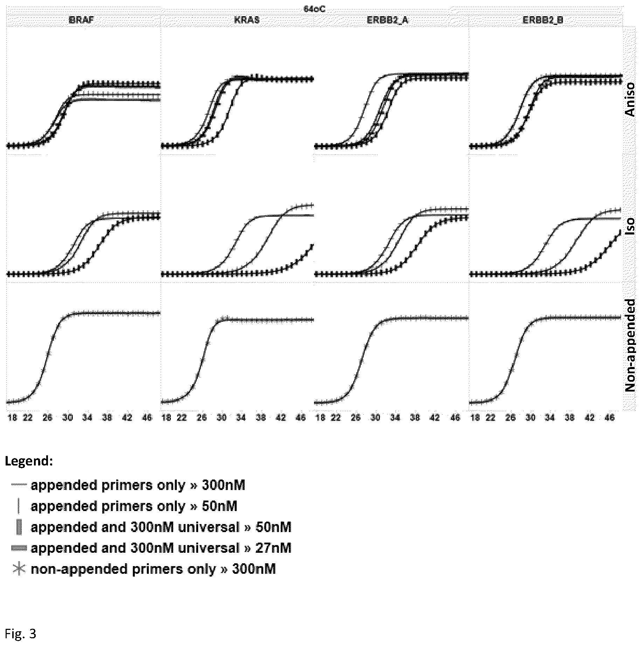

The PCR reactions were performed in 20 ul reaction volumes in a PCR buffer including the following: 10mM Tris pH8.6 @ 25oC; 2mM MgCl₂; 0.25x SYBR green final from a 100x stock in DMSO; 0.2mM dATP; 0.2mM dGTP; 0.2mM dCTP; 0.2mM dTTP; 0.2Units/µl Gotaq (Promega); HD200 & HD301: 1/1 mix of horizon FFPE liquefacts as input DNA (many genomes, not clonal, and reasonably fragmented) 0.5ng per PCR. Individual mixes further included variable amounts of target specific appended hairpin primers or non-appended control primers; the variable amounts are as follows: 300nM, 50nM, 27nM and are indicated in the PCR curves as shown in Figure 3. In the mixes including universal hairpin primers (the hairpin sequences as shown above), the each "Aniso" or "Iso" hairpin primer was used at concentration of 300nM.

The PCR profile was as follows, and the detection of amplicons was performed on FAM channel of qPCR thermocycler (BioRad).
PCR profile
2' at 95°C
followed by
2 cycles of 3" at 95°C, 60" at 64°C
followed by
38 cycles of 3" at 95°C, 30" at 64°C.

The results per amplicon and PCR mix are shown in Figure 3. As expected, they show that for some amplicons lowering the concentration of a target hairpin specific primer from 300nM to 50nM results in reduction in amplification, as seen by higher Cq values. Further lowering of the concentration to 27nM cased a visible amplification reduction for all the amplicons (not shown). Importantly, although this decrease could not be rescued by an addition of universal isotropical primers to the mix (second row), it is almost entirely rescued by an addition of the universal anisotropical hairpin primers (first row). In almost all cases, the universal anisotropical hairpin primers recovered the amplicon Cq values to the levels as seen with gene specific primers at the maximum concentration of 300nM.

The data demonstrates that the presented herein strategy allows to amplify at high amplification efficiency specific amplicons using even very low concentrations of target specific primers and a universal primer pair with anisotropical hairpins. Due to the fact that the concentration of the target specific primers can be very low, present methods allow to include potentially an even a large amount (i.e. >50 plex, > 100 plex, >250plex, >200 plex etc.) of said target specific primers in a PCR mix. Furthermore, as these target specific primers are appended with anisotropical hairpin sequences, the presence of these appended sequences allows to include unique molecular identifiers (UMI) for various NGS strategies in each target specific primer pair, e.g. within the loop or the hairpin or just before the target specific sequence of the primer. Thanks to this, massive NGS target enriching multiplex reactions can be envisaged that generate NGS-suitable libraries in one reaction tube.

## Claims

1. A method of thermocycling comprising providing at least two primer pairs in a mix, the first primer pair being a target-specific primer pair and the second pair being a universal primer pair, and subjecting the mix to a thermocycling protocol,
**the method characterised in that**
the target-specific primer pair comprises a target-specific primer comprising
a target-specific sequence having melting temperature Tₘ₋ₜₛ and
at a S'-end of said target specific sequence a first anisotropical hairpin defined as having a first loop and a first anisotropical stem comprising at least one G-T or G-U pairing and having melting temperature Tₘ₋ₐₛ₁,
wherein Tₘ₋ₐₛ₁ is at least 8°C higher than Tₘ₋ₜₛ;
and **in that**
the universal primer pair comprises a universal primer comprising a second anisotropical hairpin defined as having a second loop and a second anisotropical stem comprising at least one G-T or G-U pairing, having melting temperature Tₘ₋ₐₛ₂ and having at least 90% sequence similarity to the first anisotropical stem.

2. Method according to claim 1, wherein the thermocycling protocol comprises an annealing step at a temperature Tₐ being at least 6°C lower than Tₘ₋ₐₛ₁, and preferably being not more than 2 °C different from Tₘ₋ₜₛ.

3. Method according to claim 1 or claim 2, wherein the temperature difference between Tₐ and Tₘ₋ₜₛ is not more than 2°C, preferably not more than 1°C, most preferably wherein Tₐ is equal to Tₘ₋ₜₛ.

4. Method according to any of claims 1 to 3, wherein any of Tₘ₋ₜₛ and/or Ta is at least 8°C, preferably 9°C, most preferably 10°C lower than Tₘ₋ₐₛ₁ and Tₘ₋ₐₛ₂.

5. Method according to any of claims 1 to 4, wherein any of Tₘ₋ₜₛ and/or Ta is between 52-66°C, preferably 54-64°C, more preferably is between 56-62°C, most preferably is between around 58-68°C.

6. Method according to any of claims 1 to 5, wherein the second anisotropical stem has at least 95% sequence similarity to the first anisotropical stem, preferably being 98% sequence similarity, most preferably being identical to the first anisotropical stem (or wherein the difference between Tₘ₋ₐₛ₁ and Tₘ₋ₐₛ₂ is not more than 3°C, preferably not more than 1°C, most preferably Tₘ₋ₐₛ₁ is equal to Tₘ₋ₐₛ₂.

7. Method according to any of claims 1 to 6, wherein Tₘ₋ₐₛ₁ and preferably Tₘ₋ₐₛ₂ are at least 70°C or higher.

8. Method according to any of claims 1 to 7, wherein the thermocycling protocol further comprises an annealing step at a temperature Tₐ₂, wherein Tₐ₂ > Tₐ.

9. Method according to any of claims 1 to 8, wherein the concentration of the target-specific primer pair is at least 5 times lower than the concentration of the universal primer pair, preferably being at least 8 times lower, most preferably being at least 10 times lower.

10. Method according to any of claims 1 to 9, wherein the PCR mix comprises at least two, preferably more target-specific primer pairs.

11. Method according to any of claims 1 to 10, wherein the target-specific primer further comprises a unique molecular identifier (UMI) sequence at the S'-end of the gene-specific sequence.

12. Method according to any of claims 1 to 11, wherein the universal primer pair comprises an index or another NGS-strategy-specific barcode sequence.

13. Method according to any of claims 1 to 12, wherein the method results in NGS-ready amplicons.

14. A kit comprising a target-specific primer pair and a universal primer pair,
**the kit characterized in that**
the target-specific primer pair comprises a target-specific primer comprising
a target-specific sequence having melting temperature Tₘ₋ₜₛ and
at a S'-end of said target specific sequence a first anisotropical hairpin defined as having a first loop and a first anisotropical stem comprising at least one G-T or G-U pairing and having melting temperature Tₘ₋ₐₛ₁,
wherein Tₘ₋ₐₛ₁ is at least 8°C higher than Tₘ₋ₜₛ;
and **in that**
the universal primer pair comprises a universal primer comprising a second anisotropical hairpin defined as having a second loop and a second anisotropical stem comprising at least one G-T or G-U pairing, having melting temperature Tₘ₋ₐₛ₂ and having at least 90% sequence similarity to the first anisotropical stem.

15. A kit according to claim 14, wherein the kit comprises a cartridge engageable with an automated platform.

## Patentansprüche

1. Ein Thermocycling-Verfahren, das die Bereitstellung von mindestens zwei Primer-Paaren in einer Mischung umfasst, wobei das erste Primer-Paar ein zielspezifisches Primer-Paar ist und das zweite Paar ein universales Primer-Paar ist, sowie die Beaufschlagung der Mischung mit einem Thermocycling-Protokoll,
**wobei das Verfahren dadurch gekennzeichnet ist, dass**
das zielspezifische Primer-Paar einen zielspezifischen Primer umfasst, mit
einer zielspezifischen Sequenz mit der Schmelztemperatur Tₘ₋ₜₛ und
an einem 5'-Ende der zielspezifischen Sequenz einer ersten anisotropen Haarnadel, die durch eine erste Schlaufe und einen ersten anisotropen Stamm mit mindestens einer G-T oder G-U Paarung sowie eine Schmelztemperatur Tₘ₋ₐₛ₁ definiert ist,
wobei Tₘ₋ₐₛ₁ mindestens 8°C höher ist als Tₘ₋ₜₛ;
und **dass**
das universale Primer-Paar einen universalen Primer mit einer zweiten anisotropen Haarnadel umfasst, die durch eine zweite Schlaufe und einen zweiten anisotropen Stamm mit mindestens einer G-T oder G-U Paarung, eine Schmelztemperatur Tₘ₋ₐₛ₂ und mindestens 90% Sequenzähnlichkeit mit dem ersten anisotropen Stamm definiert ist.

2. Verfahren nach Anspruch 1, wobei das Thermocycling-Protokoll einen Glühschritt bei einer Temperatur Tₐ umfasst, die mindestens 6°C niedriger als Tₘ₋ₐₛ₁ ist und sich vorzugsweise um nicht mehr als 2°C von Tₘ₋ₜₛ unterscheidet.

3. Verfahren nach Anspruch 1 oder Anspruch 2, wobei die Temperaturdifferenz zwischen Tₐ und Tₘ₋ₜₛ nicht mehr als 2°C beträgt, vorzugsweise nicht mehr als 1°C, höchst bevorzugt wobei Tₐ gleich Tₘ₋ₜₛ ist.

4. Verfahren nach einem der Ansprüche 1 bis 3, wobei Tₘ₋ₜₛ und/oder Tₐ mindestens 8°C, vorzugsweise 9°C, höchst bevorzugt 10°C niedriger als Tₘ₋ₐₛ₁ und Tₘ₋ₐₛ₂ ist/sind.

5. Verfahren nach einem der Ansprüche 1 bis 4, wobei Tₘ₋ₜₛ und/oder Tₐ zwischen 52-66°C, vorzugsweise 54-64°C liegt, weiter bevorzugt zwischen 56-62°C liegt, höchst bevorzugt zwischen ca. 58-68°C liegt/liegen.

6. Verfahren nach einem der Ansprüche 1 bis 5, wobei der zweite anisotrope Stamm mindestens 95% Sequenzähnlichkeit mit dem ersten anisotropen Stamm hat, wobei die Sequenzähnlichkeit vorzugsweise 98% beträgt, wobei er höchst bevorzugt identisch mit dem ersten anisotropen Stamm ist (oder wobei der Unterschied zwischen Tₘ₋ₐₛ₁ und Tₘ₋ₐₛ₂ nicht mehr als 3°C, vorzugsweise nicht mehr als 1°C beträgt, höchst bevorzugt Tₘ₋ₐₛ₁ gleich Tₘ₋ₐₛ₂ ist.

7. Verfahren nach einem der Ansprüche 1 bis 6, wobei Tₘ₋ₐₛ₁ und vorzugsweise Tₘ₋ₐₛ₂ mindestens 70°C oder mehr betragen.

8. Verfahren nach einem der Ansprüche 1 bis 7, wobei das Thermocycling-Protokoll weiterhin einen Glühschritt bei einer Temperatur Tₐ₂ umfasst, wobei Tₐ₂>Tₐ.

9. Verfahren nach einem der Ansprüche 1 bis 8, wobei die Konzentration des zielspezifischen Primer-Paares mindestens fünfmal niedriger als die Konzentration des universalen Primer-Paares ist, wobei sie vorzugsweise mindestens achtmal niedriger, höchst bevorzugt mindestens zehnmal niedriger ist.

10. Verfahren nach einem der Ansprüche 1 bis 9, wobei die PCR-Mischung mindestens zwei, vorzugsweise mehr zielspezifische Primer-Paare umfasst.

11. Verfahren nach einem der Ansprüche 1 bis 10, wobei der zielspezifische Primer weiterhin eine eindeutige molekulare Kennungs- (unique molecular identifier, UMI) Sequenz an dem 5'-Ende der genspezifischen Sequenz umfasst.

12. Verfahren nach einem der Ansprüche 1 bis 11, wobei das universale Primer-Paar eine Index- oder eine andere NGS-strategiespezifische Barcode-Sequenz umfasst.

13. Verfahren nach einem der Ansprüche 1 bis 12, wobei das Verfahren zu NGS-bereiten Amplicons führt.

14. Ein Kit mit einem zielspezifischen Primer-Paar und einem universalen Primer-Paar,
**wobei das Kit dadurch gekennzeichnet ist, dass**
das zielspezifische Primer-Paar einen zielspezifischen Primer umfasst mit
einer zielspezifischen Sequenz mit einer Schmelztemperatur Tₘ₋ₜₛ und
an einem 5'-Ende der zielspezifischen Sequenz einer ersten anisotropen Haarnadel, die durch eine erste Schlaufe und einen ersten anisotropen Stamm mit mindestens einer G-T oder G-U Paarung und eine Schmelztemperatur Tₘ₋ₐₛ₁ definiert ist, wobei Tₘ₋ₐₛ₁ mindestens 8°C höher als Tₘ₋ₜₛ ist;
und **dass**
das universale Primer-Paar einen universalen Primer mit einer zweiten anisotropen Haarnadel umfasst, die durch eine zweite Schlaufe und einen zweiten anisotropen Stamm mit mindestens einer G-T oder G-U Paarung, eine Schmelztemperatur Tₘ₋ₐₛ₂ und mindestens 90% Sequenzähnlichkeit mit dem ersten anisotropen Stamm definiert ist.

15. Ein Kit nach Anspruch 14, wobei das Kit eine Patrone umfasst, die in eine automatisierte Plattform einrastbar ist.

## Revendications

1. Procédé de thermocyclage comprenant la fourniture d'au moins deux paires d'amorces dans un mélange, la première paire d'amorces étant une paire d'amorces spécifiques d'une cible et la deuxième paire étant une paire d'amorces universelles, et la soumission du mélange à un protocole de thermocyclage,
le procédé étant **caractérisé en ce que**
la paire d'amorces spécifiques d'une cible comprend une amorce spécifique d'une cible comprenant
une séquence spécifique d'une cible ayant une température de fusion Tₘ₋ₜₛ et
à une extrémité 5' de ladite séquence spécifique d'une cible, une première épingle à cheveux anisotrope définie comme ayant une première boucle et une première tige anisotrope comprenant au moins un appariement G-T ou G-U et ayant une température de fusion Tₘ₋ₐₛ₁,
dans lequel Tₘ₋ₐₛ₁ est supérieure d'au moins 8°C à Tₘ₋ₜₛ ;
et **en ce que**
la paire d'amorces universelles comprend une amorce universelle comprenant une deuxième épingle à cheveux anisotrope définie comme ayant une deuxième boucle et une deuxième tige anisotrope comprenant au moins un appariement G-T ou G-U, ayant une température de fusion Tₘ₋ₐₛ₂ et ayant au moins 90 % de similarité de séquence avec la première tige anisotrope.

2. Procédé selon la revendication 1, dans lequel le protocole de thermocyclage comprend une étape de recuit à une température Tₐ inférieure d'au moins 6°C à Tₘ₋ₐₛ₁, et de préférence différente d'au plus 2 C de Tₘ₋ₜₛ.

3. Procédé selon la revendication 1 ou la revendication 2, dans lequel la différence de température entre Tₐ et Tₘ₋ₜₛ n'est pas supérieure à 2°C, de préférence pas supérieure à 1 C, de manière préférée entre toutes dans lequel Tₐ est égale à Tₘ₋ₜₛ.

4. Procédé selon l'une quelconque des revendications 1 à 3, dans lequel l'une quelconque parmi Tₘ₋ₜₛ et/ou Tₐ est d'au moins 8 C, de préférence 9 C, de manière préférée entre toutes 10 C inférieure à Tₘ₋ₐₛ₁ et Tₘ₋ₐₛ₂ .

5. Procédé selon l'une quelconque des revendications 1 à 4, dans lequel l'une quelconque parmi Tₘ₋ₜₛ et/ou Tₐ est entre 52 et 66 C, de préférence 54 et 64 C, de manière davantage préférée est entre 56 et 62 C, de manière préférée entre toutes est entre environ 58 et 68 C.

6. Procédé selon l'une quelconque des revendications 1 à 5, dans lequel la deuxième tige anisotrope présente au moins 95 % de similarité de séquence avec la première tige anisotrope, de préférence 98 % de similarité de séquence, de manière préférée entre toutes est identique à la première tige anisotrope (ou dans lequel la différence entre Tₘ₋ₐₛ₁ et Tₘ₋ₐₛ₂ n'est pas supérieure à 3 C, de préférence pas supérieure à 1 C, de manière préférée entre toutes Tₘ₋ₐₛ₁ est égale à Tₘ₋ₐₛ₂.

7. Procédé selon l'une quelconque des revendications 1 à 6, dans lequel Tₘ₋ₐₛ₁ et de préférence Tₘ₋ₐₛ₂ sont d'au moins 70 C ou plus.

8. Procédé selon l'une quelconque des revendications 1 à 7, dans lequel le protocole de thermocyclage comprend en outre une étape de recuit à une température Tₐ₂, dans lequel Tₐ₂ > Tₐ.

9. Procédé selon l'une quelconque des revendications 1 à 8, dans lequel la concentration de la paire d'amorces spécifiques d'une cible est au moins 5 fois inférieure à la concentration de la paire d'amorces universelles, de préférence au moins 8 fois inférieure, de manière préférée entre toutes au moins 10 fois inférieure.

10. Procédé selon l'une quelconque des revendications 1 à 9, dans lequel le mélange PCR comprend au moins deux, de préférence plusieurs paires d'amorces spécifiques d'une cible.

11. Procédé selon l'une quelconque des revendications 1 à 10, dans lequel l'amorce spécifique d'une cible comprend en outre une séquence d'identification moléculaire unique (UMI) à l'extrémité 5' de la séquence spécifique d'un gène.

12. Procédé selon l'une quelconque des revendications 1 à 11, dans lequel la paire d'amorces universelles comprend une séquence d'index ou une autre séquence de code à barres spécifique d'une stratégie NGS.

13. Procédé selon l'une quelconque des revendications 1 à 12, dans lequel le procédé fournit des amplicons prêts pour un NGS.

14. Kit comprenant une paire d'amorces spécifiques d'une cible et une paire d'amorces universelles,
le kit étant **caractérisé en ce que**
la paire d'amorces spécifiques d'une cible comprend une amorce spécifique d'une cible comprenant
une séquence spécifique d'une cible ayant une température de fusion Tₘ₋ₜₛ et
à une extrémité 5' de ladite séquence spécifique d'une cible, une première épingle à cheveux anisotrope définie comme ayant une première boucle et une première tige anisotrope comprenant au moins un appariement G-T ou G-U et ayant une température de fusion Tₘ₋ₐₛ₁,
dans lequel Tₘ₋ₐₛ₁ est supérieure d'au moins 8 C à Tₘ₋ₜₛ ;
et **en ce que**
la paire d'amorces universelles comprend une amorce universelle comprenant une deuxième épingle à cheveux anisotrope définie comme ayant une deuxième boucle et une deuxième tige anisotrope comprenant au moins un appariement G-T ou G-U, ayant une température de fusion Tₘ₋ₐₛ₂ et ayant au moins 90 % de similarité de séquence avec la première tige anisotrope.

15. Kit selon la revendication 14, dans lequel le kit comprend une cartouche pouvant être insérée dans une plate-forme automatisée.
